# EUROPÄISCHE PATENTSCHRIFT

(11) **EP 0 950 411 B1**
(45) Veröffentlichungstag und Bekanntmachung des Hinweises auf die Patenterteilung: **02.08.2006**
(21) Anmeldenummer: 99104597.2
(22) Anmeldetag: 08.03.1999
(51) Int. Cl.: A61K 31/35, A61K 31/37, A61P 3/10

(54) **Verfahren zur Herstellung eines Extraktes aus Copalchirinde und Verwendung der Neoflavonoide zur Behandlung von Diabetes**
Process of manufacturing an extract from the Copalchi stem barks and the use of neoflavonoids for the treatment of diabetes
Procédé pour la préparation d'un extrait d'écorce de Copalchi et l'utilisation des néoflavonoides pour le traitement des diabetes

(30) Priorität: 14.04.1998 DE 19816492
(43) Veröffentlichungstag der Anmeldung: 20.10.1999
(73) Patentinhaber: Gehrlicher GmbH & Co. KG, Pharmazeutische Extrakte, 82547 Eurasburg/Obb. (DE)
(72) Erfinder: Sensch, Karl Heinz, Dr., 82335 Berg/Aufkirchen (DE); Zoukas, Thomas, Dr., 81475 München (DE)
(74) Vertreter: Grünecker, Kinkeldey, Stockmair & Schwanhäusser Anwaltssozietät

(56) Entgegenhaltungen:
- G. REHER, LJ. KRAUS ET AL.: "A neoflavonoid from Coutarea Hexandra (Rubiaceae)" PHYTOCHEMISTRY, Bd. 22, Nr. 6, 1983, Seiten 1524-1525, XP002110486
- G. REHER, L. KRAUS: "New Neoflavonoids from Coutarea Latiflora" J. NAT. PROD., Bd. 47, Nr. 1, 1984, Seiten 172-174, XP002900058
- M. T. REGUERO, R. MATA ET AL: "Chemical studies on mexican plants used in traditional medicine. Part II: Cucurbitacins from Hintonia Latiflora" J. NAT. PROD. (LLOYDIA), Bd. 50, Nr. 2, 1987, Seiten 315-316, XP002110487
- A. PINTO ET AL.: "Experimental animal studies on the hypoglycemic effects of a Copalchi extract" ARZNEIMITTEL-FORSCHUNG/DRUG RES., Bd. 47(II), Nr. 7, Juli 1997 (1997-07), Seiten 829-833, XP002110488

## Beschreibung

Die Erfindung betrifft ein Verfahren zur Herstellung eines Extrakts aus Copalchirinde, und die Verwendung der Neoflavonoide des Copalchirinde zur Herstellung eines Arzneimittels zur Behandlung von Diabetes Typ II.

Erstmals 1825 fand der Name Copalchi durch Mercadieu Eingang in die europäische Literatur. Er beschrieb eine Rinde, die als Fiebermittel und in der mexikanischen Volksmedizin ähnlich der bekannten China-Rinde verwendet wurde. Seit dem Erscheinen der Copalchi-Rinden in der Literatur bestand große Unklarheit darüber, welche Pflanzen nun als eigentliche Stammpflanzen zu betrachten seien, da die Bezeichnung Copalchi sich auf Pflanzen bezog, die unterschiedlichen Familien angehören.

Heute kennt man folgende Stammpflanzen von Copalchirinden:

### Rubiaceae

Exostema caribaeum (Jacq.) Roem & Schult.
E. floribundum R. & Sch.
Hintonia (Coutarea) latiflora (Sesse & Moc. ex DC) Bullock
Coutarea hexandra (Jacq.) Schum.
C. pterosperma Standl.

Anfang des 19. Jahrhunderts erschien in Europa die erste Arbeit über eine Rinde, die unter dem Namen "Copalchi" von mittel- und südamerikanischen Eingeborenen anstelle von Chinarinde gegen Malaria verwendet wurde. Bis Anfang des 20. Jahrhunderts beschäftigten sich dann zahlreiche Autoren mit der pharmakognostischen Beschreibung der aus Mittelamerika nach Europa gelangten "Copalchi"-Rinden sowie dem Versuch einer botanisch-systematischen Zuordnung. Auffällig dabei ist die Vielfalt der angegebenen Stammpflanzen, die hauptsächlich zwei Familien, den Rubiaceae und Euphorbiaceae, zugeordnet werden können.

1913 wurde von Terres und Landa neben der Anwendungsmöglichkeit der Copalchirinde von Hintonia (Coutarea) latiflora DC. (Rubiaceae) als Malariamittel erstmals auch eine antidiabetische Wirkung beschrieben, die die Autoren im Instituto Medico Nacional in Mexico bei klinischen Versuchen beobachtet hatten.

1955 bzw. 1961 bestätigte Geyer durch tierexperimentelle Untersuchungen die blutzuckersenkende Wirkung von Extrakten aus der Rinde von Coutarea latiflora DC.

Aufgrund dieser Ergebnisse befindet sich auch heute noch ein wäßrig-alkoholischer Extrakt aus der Rinde von Hintonia (Coutarea) latiflora DC. mit einem Coutareageningehalt von 0,1 bis 0,5 m/v % unter dem Namen Sucontral^{R} als Antidiabetikum im Handel. Arzneimittelforschung/Drug Res. 47-11 (7), Juli 1997, Seiten 829-833 offenbart eine Untersuchung des blutzuckersenkenden Effekts eines Copalchi-Extrakts.

Man unterscheidet verschiedene Formen des Diabetes mellitus. Der Typ-I-Diabetes (juveniler Diabetes, Insulin-abhängiger Diabetes) manifestiert sich meist im jugendlichen Alter und ist gekennzeichnet durch eine Mangel an Insulin. Als Ursache des Insulin-Mangels werden Autoimmunprozesse und Virusinfektionen des Pankreas auf dem Hintergrund einer genetischen Disposition diskutiert. Dagegen tritt der Typ-II-Diabetes (Alters-Diabetes, Insulin-unabhängiger Diabetes) in der Regel bei älteren, meist übergewichtigen Menschen auf. Dabei wird eine herabgesetzte Ansprechbarkeit der Erfolgsorgane auf das Insulin bei normalem oder erhöhtem Blutinsulinspiegel angenommen. Diese Form des Diabetes ist erblich (Römpp Lexikon Chemie, 10. Auflage, Bd. 2, Seite 930, (1997).

Die Extrakte der Colpalchirinde enthalten eine Vielzahl von Substanzen, beispielsweise Alkaloide, Neoflavonoide, Cucurbitacine, polyphenolische Substanzen und Saponine. Von diesen wurden beispielsweise das Neoflavonoid Coutareagenin identifiziert. Coutareagenin ist 5-Hydroxy-7-methoxy-4-(3,4-dihydroxyphenyl)-2H-benzo-1-pyran-2-on der folgenden Struktur:

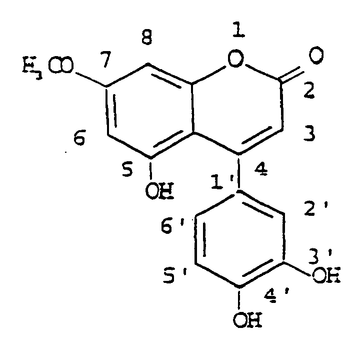

Diese Substanz kann nach der Methode von linuma, Phytochemistry 26 (11), 3096 (1987) synthetisiert werden. Phytochemistry 22(6), 1983, seiten 1524-1525 offenbart Extrakte aus Coutarea. J. Nat. Prod., 47(1), 1984, 172-174 offenbart die Isolierung von Stoffen aus Coutarea latiflora. J. Nat. Prod. (LCoydia) offenbart die Isolierung von Cucurbitacinen aus Hintonia latiflora Die Vielzahl der Bestandteile der Copalchiextrakte hat es bislang unmöglich gemacht, eine Dosierung dieses Mittels für die verschiedenen zu behandelnden Erkrankungen anzugeben, da nicht bekannt war, welches die wirksame(n) Komponente(n) ist (sind).

Es war also die Aufgabe des vorliegen den Erfindung bessere Erkenntnisse über die wirksamen Bestandteils des Copalchirinde zu gewinnen.

Überraschenderweise wurde gefunden, daß die Neoflavonoide der Copalchirinde, insbesondere das Coutareagenin, die wirksame Komponente bei der Behandlung von Diabetes ist.

Da die wirksamen Komponenten der Copalchiextrakte für die Behandlung von Diabetes bestimmt worden sind, ist es nun möglich, Extrakte mit bestimmten Anteilen dieser wirksamen Komponenten herzustellen und also ein definiertes Extraktionsverfahren zu entwickeln.
Diese wirksamen Komponenten sind Neoflavonoide, d.h. natürlich vorkommende Substanzen, die strukturell und biogenetisch mit den Flavonoiden und Isoflavonoiden verwandt sind. Repräsentative Beispiele dafür sind die 4-Phenylcumarinderivate, dargestellt durch eine der Strukturen 1 oder 2 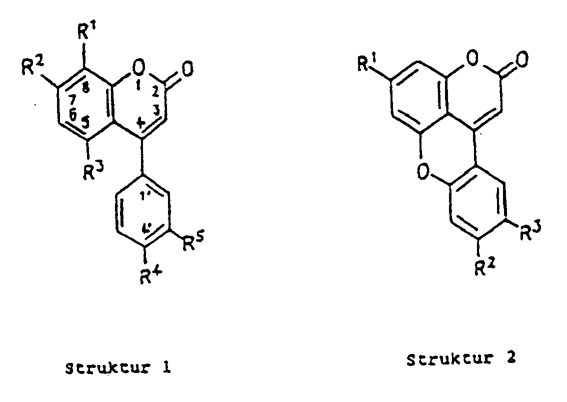 worin R¹ und R⁵ H, OH oder OCH₃ bedeuten, R² und R⁴OH oder OCH₃ bedeuten und R³ OH, OCH₃ oder ein glykosidischer Rest ist; und R⁴ und R⁵ miteinander zu einer -O-CH₂-O-Gruppe verbunden sein können. Beispiele für Verbindungen der Struktur 1 oder 2 sind in den folgenden Tabellen angegeben.

Die vorliegende Erfinden, stellt also das Verfahren nach Anspruch 1 und die Verwendung nach Anspruch 5 zur Verfingung Bevorzugte Ausführungsformen sind in der Unteransprüchen angegeben.

**Tab. 1: Bisher isolierte Neoflavonoide mit der Struktur 1 aus Coutarea hexandra**

| R¹ | R² | R³ | R⁴ | R⁵ |
|---|---|---|---|---|
| H | OCH₃ | OH | OH | OH |
| H | OCH₃ | OCH₃ | OCH₃ | H |
| H | OCH₃ | OCH₃ | OH | H |
| H | OCH₃ | OCH₃ | OCH₃ | OH |
| H | OCH₃ | OCH₃ | OH | OH |
| H | OCH₃ | OCH₃ | O-CH₂- | -O |
| H | OCH₃ | O-β-D-Glucose | OH | OH |
| H | OH | O-β-D-Glucose | OH | OH |
| H | OH | 5-O-β-D-Apiose-(1->6)-β-D-Glucose | OH | OH |
| H | OCH₃ | 5-O-β-D-Xylose-(1->6)-β-D-Glucose | OH | OH |
| OH | OCH₃ | OCH₃ | OH | OH |
| OH | OCH₃ | OCH₃ | OCH₃ | OCH₃ |
| OCH₃ | OCH₃ | OCH₃ | OCH₃ | OH |
| OH | OCH₃ | OCH₃ | OCH₃ | OH |
| OH | OCH₃ | OCH₃ | OH | OCH₃ |

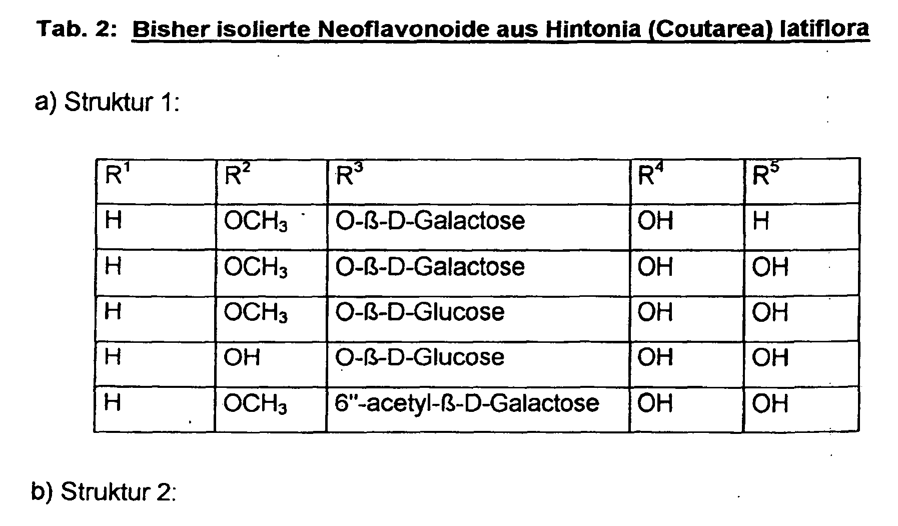

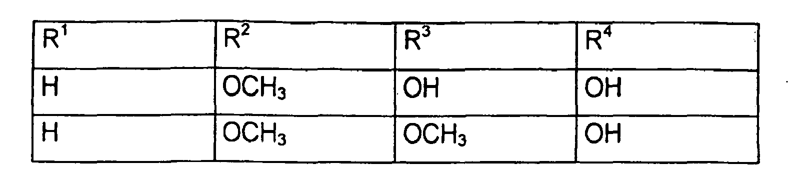

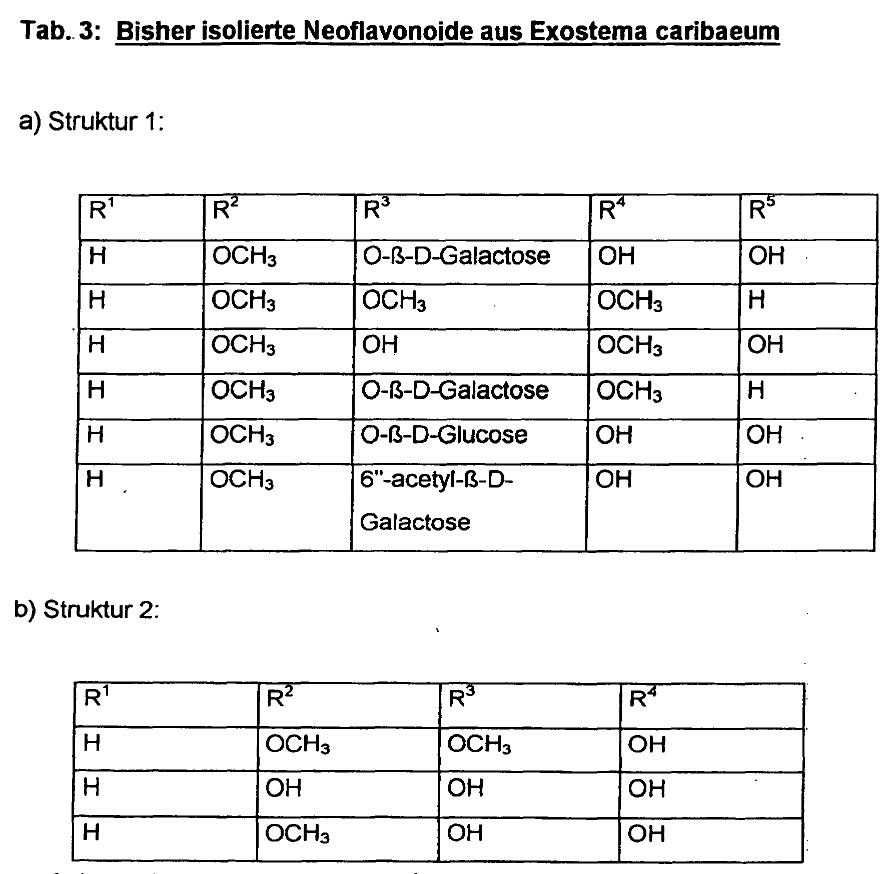

Ein im Rahmen der Erfindung gemäß Anspruch 5 insbesondere bevorzugtes Neoflavonoid der Copalchirinde ist das Coutareagenin.

Das erfindungsgemäße Verfahren kann wie folgt durdgeführt werden.

Gemäß dem ersten erfindungsgemäßen Verfahren wird Copalchirinde in einem ersten Extraktionsschritt mit einem wäßrigen Lösungsmittel extrahiert, das Extraktionsmittel anschließend abgetrennt und das so erhaltene Produkt in einem zweiten Extraktionsschritt mit mindestens einem lipophilen Lösungsmittel erneut extrahiert. Anschließend können die flüchtigen Komponenten des Extrakts abgetrennt werden, wenn dies erforderlich ist.

Vorzugsweise wird der Extrakt nach dem ersten Extraktionsschritt mit einer Säure hydrolysiert. Bevorzugte Säuren sind physiologisch verträgliche, nicht oxidierende Säuren. Beispiele hierfür sind Salzsäure, Phosphorsäure, Dihydrogenphosphate, Alkylmonocarbonsäuren, z.B. Essigsäure oder Propionsäure, verdünnte Schwefelsäure, stark saure lonenaustauscher und Polycarbonsäuren, wie Zitronensäure, Oxalsäure und Bemsteinsäure.

Als Lösungsmittel für den ersten Extraktionsschritt können beispielsweise Mischungen von Wasser mit mindestens einem organischen Lösungsmittel, gewählt aus Alkoholen, wie Ethanol und Methanol, Essigsäureestern, wie Ethylacetat und Methylacetat, Aceton und Propylenglycol, verwendet werden. Der Wasseranteil kann im Bereich von 4 bis 95 m/v % liegen.

Das lipophile Lösungsmittel für den zweiten Extraktionsschritt kann beispielsweise gewählt werden aus Atkoholen, wie Ethanol und Methanol, Essigsäureestem, wie Ethylacetat und Methylacetat, Aceton, Propylenglycol, Polyethylenglycol 300 bis 6000, chlorierten Kohlenwasserstoffen, wie Dichlormethan und Chloroform, und Mischungen davon.

Der erfindungsgemäß hergestellte Extrakt ist im Hinblich auf die Neoflavonoide angereichst. Er enthält mindestens ein Neoflavonoid, wobei des Gehalt an Coutareagenin bevorzugt auf 0,6 bis 99 m/v%, starkes bevorzugt 2 bis 95 m/v %, insbesondere bevorzugt 5 bis 90 m/v % eingestellt wird.

In den erfindungsgemäßen Verfahren können bekannte Extraktionsverfahren eingesetzt werden. Beispiele sind Flüssig-flüssig-Extraktion und Flüssig-fest-Extraktion, die üblicherweise zur Extraktion von Drogen verwendet werden. Die Extraktionen können als ruhende Mazeration, Bewegungsmazeration, Wirbelextraktion, Digestion, Perkolation, Reperkolation, Evakolation und Diakolation, Kombinationen von Mazeration und Perkolation, Ultraschallextraktion, Gegenstromextraktion, Extraktionen mit Separatoren, Zentrifugen und Dekantem ausgestaltet sein.

Erfindungsgemäß werden der erfindungsgemäß hergestellte Extrakt aus Copalchirinde oder die Neoflavonoide der Copalchirinde als Arzneimittel, vorzugsweise zur Behandlung von Diabetes, insbesondere Diabetes Typ II, eingesetzt.

Dazu können die Neoflavonoide der Copalchirinde, insbesondere das Coutareagenin, einzeln oder als Gemisch verwendet werden.

Die Neoflavonoide, insbesondere das Coutareagenin, können dazu einzeln oder als Gemisch nach üblichen Verfahren aus Copalchirinde isoliert oder nach bekannten Verfahren synthetisch hergestellt werden.

Der erfindungsgemäße Extrakt, sowie die Neoflavonoide, können als solche oder in Form von üblichen pharmazeutischen Zubereitungen, wie Kapseln, Tabletten, Dragees, Granulaten, Tinkturen, Fluid-, Siccum-, Spissum- oder Oleosum-Extrakte oder Mikrokapseln verabreicht werden.

Dem erfindungsgemäß hergestellten Extrakt können weitere Wirkstoffe, wie Mineralien und Vitamine, unbedenkliche bzw. annehmbare natürliche oder synthetische Zusätze oder Hilfsstoffe, wie Bindemittel, Sprengmittel, Gleitmittel, Trennmittel, Lösungsmittel, Stabilisatoren, Färbemittel und Geschmackskorrigentien zugesetzt werden, die in Abhängigkeit von der gewünschten Darreichungsform ausgewählt werden. Beispiele für erfindungsgemäß verwendbare Hilfsstoffe sind
- Bindemittel, wie Stärke, Alginate, Gelatine, Zucker, Johannisbrotkernmehl, Cellulosederivate, wie Celluloseether, und Polymere, wie Polyvinylpyrrolidon;
- Sprengmittel, wie Stärke und Stärkeether;
- Gleit- und Trennmittel, wie Talkum, Stearate, wie Calcium- und Magnesiumstearat, Magnesium- und Calciumcarbonat, Cellulose, Magnesiumoxid, kolloidale Kieselsäure, Silikate, wie Natrium-, Magnesium-, Calcium- und Aluminiumsilikat, Trennmehle, wie Brotmehl, Getreideschalen-, Kartoffel-walz-, Buchweizen- und Holzmehl und Johannisbrotkemmehl;
- Lösungsmittel, wie Wasser, Alkohol und Lösungen von Bindemittein;
- Stabilisatoren, wie Fette, Öle, Aromastoffe und Stärkederivate;
- Färbemittel, wie wie lebensmittel-, und arzneimittelrechtlich zulässige natürliche und synthetische Farbstoffe und Pigmente, beispielsweise Caroten, Zuckercouleur, Betanin und Lycopin; und
- Geschmackskorrigentien, wie Gewürze, Salze, Süßungsmittel und Aromastoffe.

Die genannten Hilfsstoffe eignen sich insbesondere zum Tablettieren und Granulieren.

Die erfindungsgemäß hergestellten Extrakte, sowie Lösungen der Neoflavonoide in pharmazeutisch akzeptablen Lösungsmitteln, können auch als Injektionslösungen formuliert werden.

Die folgenden Beispiele erläutern die Erfindung;

### Beispiele

### Coutareagenin-Formulierungen

Synthetisches Coutareagenin wurde in Aqua Purificata, Propylenglycol, Macrogol 300 oder Natriumbicarbonatlösung suspendiert oder gelöst, um eine applizierbare Formulierung zu erhalten, die als solche oder in Trinkwasser aufgenommen verwendet wurde.

### Copalchiextrakt-Formulierungen

Copalchirinde wurde mit 20 bis 35% Ethanol extrahiert und der Extrakt vorsichtig eingeengt. Das erhaltene Konzentrat wurde erneut extrahiert und so ein Extrakt mit einem Gehalt an Coutareagenin von 4,52 m/v % erhalten. Als Extraktionsmittel für die zweite Extraktion wurde Propylenglykol (50% in Wasser), Macrogol 300 (50% in Wasser) oder Natriumbicarbonatlösung verwendet.

Als Blindprobe wurden auch die Lösungsmedien Propylenglykol bzw: Macrogol 300 getestet.

### Versuchstiere

Die verwendeten Versuchstiere waren Wistar Ratten des Inzuchtstammes F>28, welche unter GLP Bedingungen gehalten wurden.

Diabetes mellitus wurde bei den Ratten mit Streptozotocin (65mg/kg i.v.), 2 Wochen vor Versuchsbeginn induziert. Die Blutzuckerwerte lagen um 20mmol/l.

Den Ratten wurde nach dem Experiment 1-2 IE/kg schnell- und kurzwirkendes Insulin/regular/s.c. injiziert um sie am Leben zu halten.

Die Applikation von Propylenglykol 50%, Macrogol 300 50%, mit Coutareagenin oder Copalchi-Extrakt erfolgte intragastral per Sonde.

Copalchi-Extrakt in Natriumbicarbonat-Lösung wurde in einem Versuch über das Trinkwasser verabreicht.

Es wurde jeweils 1 Ratte pro Käfig gehalten, sie wurden ad libitum gefüttert und der Wasserverbrauch wurde 3 x täglich überprüft.

Die Versuche begannen jeweils morgens zwischen 8 und 9 Uhr.

Alle Versuchstiere fasteten nach Beginn des Versuches bis zur letzten Blutentnahme. Ein weiterer Versuch wurde mit Ratten durchgeführt, die 14 h vor Versuchsbeginn gefastet hatten.

### Geprüfte Parameter

Die Blutzuckerwerte wurden in mmol/l gemessen und dokumentiert. Kontrolliert wurden ferner die Futter- und Wasseraufnahme sowie Urinausscheidung. Das Körpergewicht der Ratten wurde täglich, oder wenigstens 2 mal in der Woche bestimmt.

Die Bestimmung des Blutzuckerspiegels erfolgte mittels der GOD-POD-Chromogen-Methode nach Korec, Experimental and Spontaneons Diabetes Mellitus in the Rat and Mouse, Edition Center of University P.J. Safarik, Kosice, CSFR (1991). Dazu wurden 5 µl Blut vom Schwanz entnommen.

Die Ergebnisse sind in den folgenden Tabellen, einschließlich der Apptikationsart der Dosierung, aufgeführt.

### Versuchsergebnisse

### Lösungsmedien

**Propylenglykol** 50%, 2ml/kg intragastral

**Versuchsreihe 1: 30 Ratten**

| Zeit in h | 0 | 2 | 4 |
|---|---|---|---|
| Blutzucker mmol/l | 20,5 | 21,8 | 21,4 |
| Blutzuckersenkunggegenüber Ausgangswert bei 0h | -- | (Erhöhung + 6%) | (Erhöhung +4%) |

**Versuchsreihe 2: 12 Ratten (f.)**

| Zeit in h | 0 | 2 | 4 |
|---|---|---|---|
| Blutzucker mmol/l | 18,4 | 19,0 | 19,0 |
| Blutzuckersenkung gegenüber Ausgangswert 0h | -- | (Erhöhung + 3%) | (Erhöhung + 3%) |

**Versuchsreihe 3: 6 Ratten (m.)**

| Zeit in h | 0 | 2 | 4 |
|---|---|---|---|
| Blutzucker mmol/l | 18,6 | 20,0 | 21,2 |
| Blutzuckersenkung gegenüber Ausgangswert 0h | -- | (Erhöhung + 8%) | (Erhöhung + 14%) |

**Macrogol 300 50%, 2ml/kg intragastral, 9 Ratten**

| Zeit in h | 0 | 2 | 4 |
|---|---|---|---|
| Blutzucker mmol/l | 14,3 | 14,6 | 14,2 |
| Blutzuckersenkung gegenüber Ausgangswert 0h | -- | (Erhöhung + 2%) | - 1 % |

### Applikation des Copalchi-Nativextrakts im Trinkwasser

**Copalchi-Nativextrakt** 1 mg/ml H₂O•NaHCO₃ im Trinkwasser, bei unveränderter Futteraufnahme. Trinkwasseraufnahme: >100ml pro Tag.

**Versuchsreihe 1: 9 Ratten**

| Datum | 27.05.96 | 28.05.96 | 29.05.96 | 30.05.96 |
|---|---|---|---|---|
| Blutzucker mmol/l | 25,3 ± 3,5 | 23,9 ± 5,4 | 19,9 ± 2,9 | 21,4 ± 3,1 |
| Blutzuckersen kung gegenüber Ausgangswert 0d | -- | -6% | -21 % | -15 % |

**Versuchsreihe 2: Copalchi-Nativextrakt 1 mg/ml im Trinkwasser: Verabreichung an 7 Ratten (m.) 1 Woche lang (vom 15.10.morgens - 21.10. morgens)**

| Datum | 15.10. | 16.10. | 17.10. | 18.10. | 21.10. | 22.10. |
|---|---|---|---|---|---|---|
| Blutzucker mmol/l | 19,9 | 19,6 | 22,6 | 12,0 | 15,1 | 21,7 |
| Blutzuckersenkung gegenüber Ausgangswert 0d | -- | -2% | (Erhöhung + 14% | 40% | -24% | *(Erhöhung* + *9%*) |

**Versuchsreihe 3: Copalchi-Nativextrakt im Trinkwasser: 6 Ratten (f.), Verabreichung 1 Woche lang (vom 15.10. morgens - 18.10. morgens)**

| Datum | 15.10. | 16.10. | 17.10. | 18.10. | 21.10. | *22.10.* |
|---|---|---|---|---|---|---|
| Blutzucker mmol/l | 18,8 | 14.8 | 12,2 | 17,7 | 12,0 | 19,2 |
| Blutzuckersenkung gegenüber Ausgangswert 0d | -- | - 21% | -35% | - 6% | - 36% | *(Erhöhung* + *2%)* |

### Copalchi-Nativextrakt in Propylenglykol 50%, intragastral

### 100 mg/kg Copalchi-Nativextrakt

**Versuchsreihe 1: 6 Ratten (f.)**

| Zeit in h | 0 | 2 | 4 |
|---|---|---|---|
| Blutzucker mmol/l | 21,6 | 22,5 | 13,8 |
| Blutzuckersenkung gegenüber Ausgangswert 0h | -- | (Erhöhung +4%) | - 36% |

**Versuchsreihe 2: Copalchi Nativextrakt 100 mg/kg, 5 Ratten (m.)**

| Zeit in h | 0 | 2 | 4 |
|---|---|---|---|
| Blutzucker mmol/l | 20,8 | 14,0 | 16,5 |
| Blutzuckersenkunggegenüber Ausgangswert 0h | -- | - 32 | - 19 |

### 200 mg/kg Copalchi-Nativextrakt

**Versuchsreihe 1: 5 Ratten (f.)**

| Zeit in h | 0 | 2 | 4 |
|---|---|---|---|
| Blutzucker mmol/l | 29,1 | 24,6 | 17,3 |
| Blutzuckersenkung gegenüber Ausgangswert 0h | -- | - 15% | - 42% |

**Versuchsreihe 2: Copalchi Nativextrakt 200 mg/kg, 5 Ratten (m.)**

| Zeit in h | 0 | 2 | 4 |
|---|---|---|---|
| Blutzucker mmol/l | 29,9 | 25,1 | 18,0 |
| Blutzuckersenkung gegenüber Ausgangswert 0h | -- | -16 % | - 40% |

### Coutareagenin in 50% Propylenglykol, intragastrale Applikation

### 3mg/kg Coutareagenin

**Versuchsreihe 1: 24 Ratten**

| Zeit in h | 0 | 2 | 4 |
|---|---|---|---|
| Blutzucker mmol/l | 23,7 | 23,8 | 19,5 |
| Blutzuckersenkung gegenüber Ausgangswert 0h | -- | (Erhöhung + 4%) | - 18% |

**Versuchsreihe 2: Coutareagenin 3 mg/kg, 12 Ratten (f.)**

| Zeit in h | 0 | 2 | 4 |
|---|---|---|---|
| Blutzucker mmol/l | 20,6 | 20,4 | 17,0 |
| Blutzuckersenkung gegenüber Ausgangswert 0h | -- | - 1 % | - 18% |

**Versuchsreihe 3: Coutareagenin 3 mg/kg, 6 Ratten (m.)**

| Zeit in h | 0 | 2 | 4 |
|---|---|---|---|
| Blutzucker mmol/l | 27,0 | 27,4 | 21,0 |
| Blutzuckersenkung gegenüber Ausgangswert 0h | -- | (Erhöhung + 1%) | - 22% |

### 9mg/kg Coutareagenin/50% Propylenglycol

**Versuchsreihe 1: 24 Ratten**

| Zeit in h | 0 | 2 | 4 |
|---|---|---|---|
| Blutzucker mmol/l | 23,8 | 20,4 | 18,1 (nach 6h:16,2) |
| Blutzuckersenkung gegenüber Ausgangswert 0h | -- | - 14% | - 24% (nach 6h: -32% |

**Versuchsreihe 2: 9mg/kg Coutareagenin/50% Propylenglycol, 7 Ratten**

| Zeit in h | 0 | 2 | 4 |
|---|---|---|---|
| Blutzucker mmol/l | 33,5 | 27,5 | 24,8 |
| Blutzuckersenkung gegenüber Ausgangswert 0h | -- | - 19% | -27% |

**Versuchsreihe 3: 9 mg/kg Coutareagenin, 12 Ratten (f.)**

| Zeit in h | 0 | 2 | 4 |
|---|---|---|---|
| Blutzucker mmol/l | 20,3 | 18,0 | 15,0 |
| Blutzuckersenkunggegenüber Ausgangswert 0h | -- | - 11% | - 26% |

**Versuchsreihe 4: 9 mg/kg Coutareagenin/50% Propylenglycol, 6 Ratten (m.)**

| Zeit in h | 0 | 2 | 4 |
|---|---|---|---|
| Blutzucker mmol/l | 26,4 | 23,5 | 21,4 |
| Blutzuckersenkunggegenüber Ausgangswert 0h | -- | - 11 % | -19% |

### 18mg/kg Coutareagenin/50% Propylenglycol

**Versuchsreihe 1: 12 Ratten**

| Zeit in h | 0 | 2 | 4 |
|---|---|---|---|
| Blutzucker mmol/l | 22.9 | 21,8 | 16,7 (nach 6h: 16,6) |
| Blutzuckersenkung gegenüber Ausgangswert 0h | -- | -5% | - 27% (nach 6h: - 27%) |

**Versuchsreihe 2: 18 mg/kg Coutareagenin, 12 Ratten (f.)**

| Zeit in h | 0 | 2 | 4 |
|---|---|---|---|
| Blutzucker mmol/l | 19,0 | 18,5 | 14,4 |
| Blutzuckersenkung gegenüber Ausgangswert Oh | -- | - 3% | - 24% |

**Versuchsreihe 3: 18 mg/kg Coutareagenin, 6 Ratten (m.)**

| Zeit in h | 0 | 2 | 4 |
|---|---|---|---|
| Blutzucker mmol/l | 26,0 | 25,2 | 18,8 |
| Blutzuckersenkunggegenüber Ausgangswert 0h | -- | -3% | - 28% |

### Coutareagenin in 50% Macrogol 300, intragastral

### 1 mg/kg Coutareagenin

**9 Ratten, die vor Versuchsbeginn 14 h gefastet haben**

| Zeit in h | 0 | 2 | 4 |
|---|---|---|---|
| Blutzucker mmol/l | 14,3 | 12,6 | 10,6 |
| Blutzuckersenkung gegenüber Ausgangswert 0h | -- | - 12% | - 26% |

### 3 mg/kg Coutareagenin/50% Macrogol

**12 Ratten**

| Zeit in h | 0 | 2 | 4 |
|---|---|---|---|
| Blutzucker mmol/l | 19,8 | 17,2 | 13,4 |
| Blutzuckersenkung gegenüber Ausgangswert 0h | -- | - 13 % | - 33 % |

### Coutareagenin 9 mg/kg/50% Macrogol

**Versuchsreihe 1:9 Ratten**

| Zeit in h | 0 | 2 | 4 |
|---|---|---|---|
| Blutzucker mmol/l | 39,1 | 34,4 | 26,8 |
| Blutzuckersenkung gegenüber Ausgangswert 0h | -- | - 12% | - 31% |

**Versuchsreihe 2: 9mg/kg Coutareagenin/50% Macrogol. 9 Ratten, die vor Versuchsbeginn 14 h fasteten.**

| Zeit in h | 0 | 2 | 4 |
|---|---|---|---|
| Blutzucker mmol/l | 26,4 | 20,2 | 22,0 |
| Blutzuckersenkunggegenüber Ausgangswert 0h | -- | - 21% | - 17% |

**Versuchsreihe 3: 9 mg/kg Coutareagenin/50% Macrogol, 9 Ratten (Wiederholung des Versuchs ohne vorheriges Fasten)**

| Zeit in h | 0 | 2 | 4 |
|---|---|---|---|
| Blutzucker mmol/l | 22,3 | 18,8 | 17,3 |
| Blutzuckersenkung gegenüber Ausgangswert 0h | -- | - 16% | - 23 % |

Die Versuchsergebnisse zeigen, daß eine deutliche blutzuckersenkende Wirkung sowohl mit Coutareagenin als auch mit dem Copalchiextrakt mit einem Coutareageningehalt von 4,52 m/v % erreicht wird. Diese Versuche zeigen insbesondere, daß die in der Copalchirinde enthaltenen Neoflavonoide, insbesondere das Coutareagenin, die antidiabetischen wirksamen Komponenten der Copalchirinde sind.

## Patentansprüche

1. Verfahren zur Herstellung eines Extrakts aus Copalchirinde umfassend die Stufen
a) Extrahieren von Copalchirinde mit einem wäßrigen Lösungsmittel;
b) Abtrennen des Extraktionsmittels; und
c) Extrahieren des in Stufe b) erhaltenen Produkts mit mindestens einem lipophilen Lösungsmittel,
worin der in Stufe a) erhaltene Extrakt vor der Abtrennung des Extraktionsmittels in Stufe b) mit einer Säure hydrolysiert wird.

2. Verfahren nach Anspruch 1, worin im Anschluß an Stufe c) die flüchtigen Komponenten abgetrennt werden.

3. Verfahren nach einem der Ansprüche 1 oder 2, worin das in Stufe c) verwendete lipophile Lösungsmittel aus Ethanol, Ethylacetat, Methanol, Methylacetat, Dichlormethan, Propylenglycol, Polyethylenglycol 300 bis 6000, Aceton und Chloroform gewählt wird.

4. Verwendung der Neoflavonoide der Copalchirinde zur Herstellung eines Arzneimittels zur Behandlung von Diabetes.

5. Verwendung nach Anspruch 5, **dadurch gekennzeichnet, daß** das Neoflavonoid Coutareagenin ist.

## Claims

1. Process for the preparation of an extract of copalchi bark, comprising the steps
a) extraction of copalchi bark using an aqueous solvent;
b) separation of the extracting agent; and
c) extraction of the product obtained in step b) using at least one lipophilic solvent,
wherein the extract obtained in step a) is hydrolysed with an acid before the extracting agent is separated off in step b).

2. Process according to claim 1, wherein, following step c), the volatile components are separated off.

3. Process according to either claim 1 or claim 2, wherein the lipophilic solvent used in step c) is selected from ethanol, ethyl acetate, methanol, methyl acetate, dichloromethane, propylene glycol, polyethylene glycol 300 to 6000, acetone and chloroform.

4. Use of neoflavonoids from copalchi bark in the preparation of a medicament for treating diabetes.

5. Use according to claim 5, **characterised in that** the neoflavonoid is coutareagenin.

## Revendications

1. Procédé de préparation d'un extrait d'écorce de Copalchi, comprenant les étapes :
a) extraction de l'écorce de Copalchi avec un solvant aqueux ;
b) séparation de l'agent d'extraction ; et
c) extraction du produit obtenu à l'étape b) avec au moins un solvant lipophile,
dans lequel l'extrait obtenu à l'étape a) est hydrolysé avec un acide avant la séparation de l'agent d'extraction à l'étape b).

2. Procédé selon la revendication 1, dans lequel les composants volatils sont séparés à la suite de l'étape c).

3. Procédé selon l'une des revendications 1 ou 2, dans lequel le solvant lipophile utilisé à l'étape c) est choisi parmi l'éthanol, l'acétate d'éthyle, le méthanol, l'acétate de méthyle, le dichlorométhane, le propylèneglycol, le polyéthylèneglycol 300 à 6000, l'acétone et le chloroforme.

4. Utilisation des néoflavonoïdes de l'écorce de Copalchi pour la fabrication d'un médicament pour le traitement du diabète.

5. Utilisation selon la revendication 5, **caractérisée en ce que** le néoflavonoïde est la coutaréagénine.
